# EUROPEAN PATENT APPLICATION

(11) **EP 3 284 431 A1**
(43) Date of publication of application: **21.02.2018**
(21) Application number: 16779988.1
(22) Date of filing: 08.04.2016
(51) Int. Cl.: A61B 18/08, A61B 17/3201

(54) **MEDICAL DEVICE**

(30) Priority: 13.04.2015 JP 2015081970
(71) Applicant: Olympus Corporation, Hachioji-shi Tokyo 192-8507 (JP)
(72) Inventor: TAKEI, Yusuke, Hachioji-shi Tokyo 192-8507 (JP); TANAKA, Kazuhiro, Hachioji-shi Tokyo 192-8507 (JP); TAKASHINO, Tomoyuki, Hachioji-shi Tokyo 192-8507 (JP)
(74) Representative: Gunzelmann, Rainer
(86) International application number: PCT/JP2016/061589
(87) International publication number: WO 2016/167197

(57) **Abstract**

A medical instrument includes: a jaw which is provided with a grasping section; and a blade which is movable close to and away from the grasping section and which extends from a distal-end portion thereof to a proximal-end portion thereof, the blade having a top portion including a ridge opposed to the grasping section, and the ridge of the top portion in the proximal-end portion being sharper than the ridge of the top portion in the distal-end portion.

## Description

### Technical Field

The present invention relates to a medical instrument capable of treating a biological tissue that is being grasped.

### Background Art

For example, Jpn. Pat. Appln. KOKAI Publication No. 2001-198137 discloses a treatment instrument including a treatment section that is formed sharply in a positon of the grasping surface of one jaw as it moves closer to the grasping surface of the other jaw. The treatment section transfers heat to the grasping surface of the one jaw while a sharp portion of the grasping surface is applying pressure to a biological tissue to be cut, and then cuts the biological tissue while being coagulated (sealed).

When a biological tissue is caught by the distal-end portion of the treatment section of the treatment instrument disclosed in Jpn. Pat. Appln. KOKAI Publication No. 2001-198137, the sharp portion of the grasping surface of the one jaw applies a great pressure on a small area of the biological tissue. In other words, when a biological tissue is caught by the distal-end portion of the treatment section of the treatment instrument disclosed in Jpn. Pat. Appln. KOKAI Publication No. 2001-198137, a great pressure will be applied locally per unit area. It is thus likely that a mark of grasping will be left on a biological tissue that is grasped simply but need not be treated.

### Summary of Invention

An object of the present invention is to provide a medical instrument capable of simply grasping a biological tissue that need not be treated while inhibiting a mark of the grasping from being left and capable of treating a biological tissue, such as a large blood vessel and a large biological tissue, which cannot be cut at a time, to prevent bleeding even though they are coagulated (sealed), cut and the like more than one time.

According to one aspect of the present invention, a medical instrument includes: a jaw which is provided with a grasping section; and a blade which is movable close to and away from the grasping section and which extends from a distal-end portion thereof to a proximal-end portion thereof, the blade having a top portion including a ridge opposed to the grasping section, and the ridge of the top portion in the proximal-end portion being sharper than the ridge of the top portion in the distal-end portion.

### Brief Description of Drawings

FIG. 1 is a schematic view showing a treatment system according to first to third embodiments.
FIG. 2 is a schematic diagram showing a treatment section of a treatment instrument of the treatment system according to the first embodiment.
FIG. 3A is a schematic cross-sectional view taken along line 3A-3A in FIG. 2 and showing a biological tissue grasped between a sealing area of a blade of a first treatment piece of the treatment section of the treatment instrument according to the first embodiment and a blade rest of a second treatment piece thereof.
FIG. 3B is a schematic cross-sectional view taken along line 3B-3B in FIG. 2 and showing a biological tissue grasped between a cutting area of the blade of the first treatment piece of the treatment section of the treatment instrument according to the first embodiment and the blade rest of the second treatment piece thereof.
FIG. 4 is a schematic perspective view showing a sealing area formed in the distal-end portion of the blade of the first treatment piece of the treatment section of the treatment instrument according to the first embodiment and a cutting area formed on the proximal-end side of the sealing area.
FIG. 5A is a schematic diagram showing a biological tissue grasped between a sealing area of the blade of the treatment section of the treatment instrument according to the first embodiment and a grasping section of the blade rest when viewed from the direction of arrow 5A in FIG. 5B.
FIG. 5B is a schematic diagram showing a biological tissue grasped between the sealing area of the blade of the treatment section of the treatment instrument according to the first embodiment and the grasping section of the blade rest when viewed from the direction of arrow 5B in FIG. 5A.
FIG. 6 is a schematic perspective diagram showing a biological tissue grasped between the sealing area of the blade of the treatment section of the treatment instrument according to the first embodiment and the grasping section of the blade rest.
FIG. 7A is a schematic diagram showing a biological tissue grasped between the sealing and cutting areas of the blade of the treatment section of the treatment instrument according to the first embodiment and the grasping section of the blade rest when viewed from the direction of arrow 7A in FIG. 7B.
FIG. 7B is a schematic diagram showing a biological tissue grasped between the sealing and cutting areas of the blade of the treatment section of the treatment instrument according to the first embodiment and the grasping section of the blade rest when viewed from the direction of arrow 7B in FIG. 7A.
FIG. 8 is a schematic diagram showing a biological tissue that is cut while being sealed by the blade to which heat is transferred from a heater heated when the biological tissue is grasped as shown in FIGS. 7A and 7B.
FIG. 9A is a schematic diagram showing a blood vessel grasped between the sealing and cutting areas of the blade of the treatment section of the treatment instrument according to the first embodiment and the grasping section of the blade rest when viewed from the direction of arrow 9A in FIG. 9B.
FIG. 9B is a schematic diagram showing a blood vessel grasped between the sealing and cutting areas of the blade of the treatment section of the treatment instrument according to the first embodiment and the grasping section of the blade rest when viewed from the direction of arrow 9B in FIG. 9A.
FIG. 10 is a schematic diagram showing a blood vessel that is cut while being sealed by the blade to which heat is transferred from the heater heated when the blood vessel is grasped as shown in FIGS. 9A and 9B.
FIG. 11 is a schematic perspective view showing a sealing area formed in the distal-end portion of a blade of a first treatment piece of a treatment section of a treatment instrument according to a first modification to the first embodiment and a cutting area formed on the proximal-end side of the sealing area.
FIG. 12 is a schematic perspective view showing a non-slip portion in a sealing area formed in the distal-end portion of a blade of a first treatment piece of a treatment section of a treatment instrument according to a second modification to the first embodiment.
FIG. 13 is a schematic cross-sectional view taken along line 3A-3A in FIG. 2 and showing a treatment section of a treatment instrument of a treatment system according to a third modification to the first embodiment.
FIG. 14 is a schematic diagram showing a second treatment piece of a treatment section of a treatment instrument of a treatment system according to a fourth modification to the first embodiment, which is formed as a seesaw jaw.
FIG. 15A is a schematic cross-sectional view showing a biological tissue grasped between a sealing area of a blade of a first treatment piece of a treatment section of a treatment instrument of a treatment system according to a second embodiment and a blade rest of a second treatment piece thereof.
FIG. 15B is a schematic cross-sectional view showing a biological tissue grasped between a cutting area of the blade of the first treatment piece of the treatment section of the treatment instrument of the treatment system according to the second embodiment and the blade rest of the second treatment piece thereof.
FIG. 16 is a schematic perspective view showing the first treatment piece of the treatment section of the treatment instrument of the treatment system according to the second embodiment.
FIG. 17A is a schematic perspective view showing a modification to the first treatment piece of the treatment section of the treatment instrument of the treatment system according to the second embodiment.
FIG. 17B is a schematic cross-sectional view taken along line 3A-3A in FIG. 2 and showing a treatment section of a treatment instrument of a treatment system according to a modification to the second embodiment.
FIG. 18 is a schematic cross-sectional view showing a biological tissue grasped between a blade of a first treatment piece of a treatment section of a treatment instrument of a treatment system according to a third embodiment and a blade rest of a second treatment piece thereof.

### Description of Embodiments

Embodiments of the present invention will be described below with reference to the drawings.

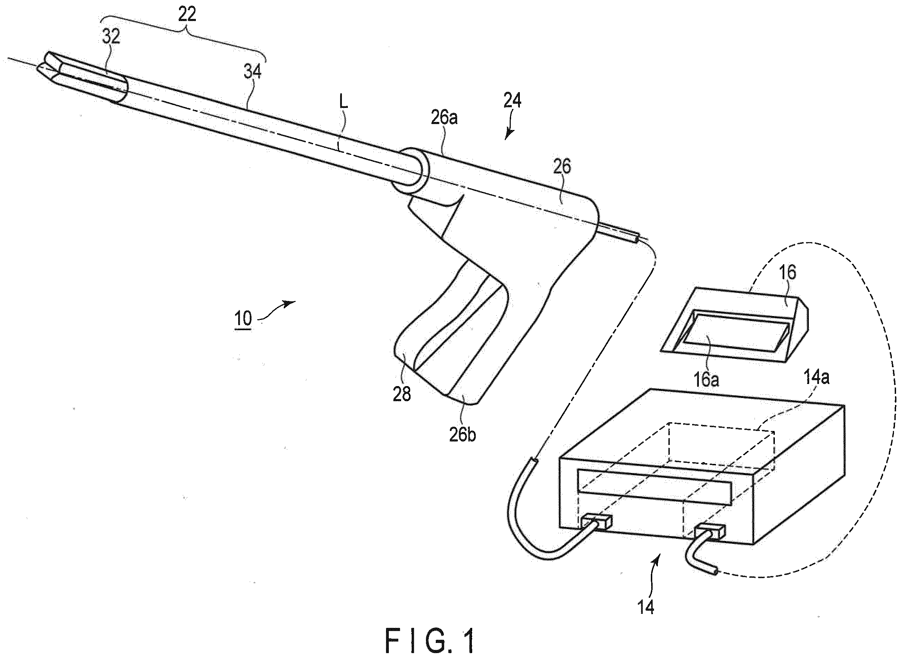

A first embodiment will be described with reference to FIGS. 1 to 10.

As shown in FIG. 1, the treatment system (energy treatment system) 10 according to this embodiment includes a treatment instrument (energy treatment instrument) 12 as a medical instrument and a controller 14 including an energy source 14a.

The treatment instrument 12 includes an insertion section 22 and an operation section 24 provided at the proximal-end portion of the insertion section 22.

The operation section 24 includes an operation section main body 26 and a knob 28 to operate a treatment section 32 (described later) of the insertion section 22. The operation section main body 26 has one end 26a and the other end 26b. The one end 26a of the operation section main body 26 is connected to the proximal end of a sheath 34 (described later) of the insertion section 22. The other end 26b of the operation section main body 26 can move close to and away from the knob 28. In FIG. 1, the operation section main body 26 is substantially L-shaped, but its shape can be selected as appropriate.

The insertion section 22 includes a treatment section 32 and a sheath 34. The treatment section 32 is provided at the distal-end portion of the sheath 34 of the insertion section 22. The sheath 34 is provided at the proximal-end portion of the treatment section 32 and is connected to the one end 26a of the operation section main body 26 of the operation section 24.

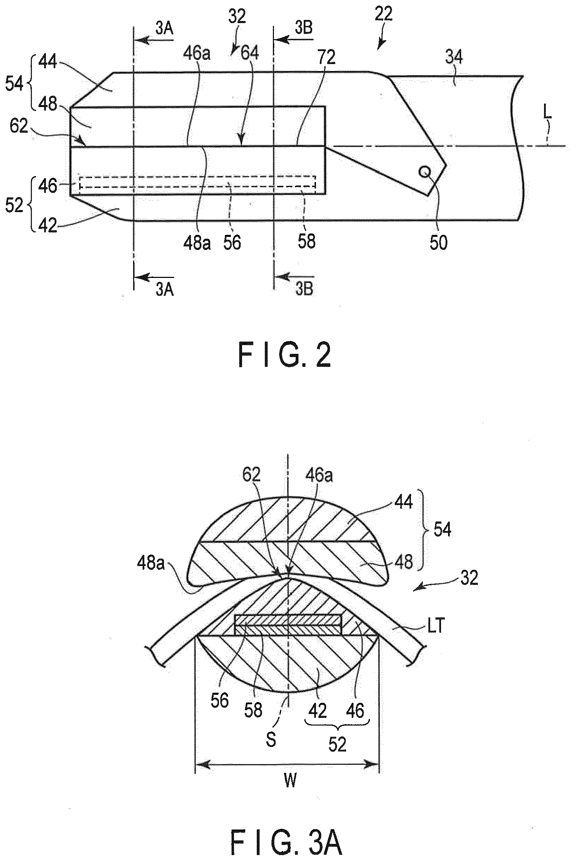

As shown in FIGS. 2 to 3B, the treatment section 32 of the insertion section 22 includes first and second jaws 42 and 44 which are opened and closed relatively by operating the operation section 24, a blade (first pressure applying member) 46 provided on the first jaw 42, and a blade rest (second pressure applying member) 48 provided on the second jaw 44.

This embodiment will be described on the assumption that the first jaw 42 is provided with the blade 46 to form a first treatment piece 52 and the second jaw 44 is provided with the blade rest 48 to form a second treatment piece 54. It is of course favorable that the first jaw 42 is provided with the blade rest 48 to form a first treatment piece 52 and the second jaw 44 is provided with the blade 46 to form a second treatment piece 54.

In FIG. 2, the first jaw 42 is fixed to the sheath 34 integrally as one unit. As the movable knob 28 moves close to the other end 26b of the operation section main body 26 shown in, e.g. FIG. 1, the second jaw 44 rotates around a pivot 50 and moves close to the first jaw 42. Moreover, as the movable knob 28 moves away from the other end 26b of the operation section main body 26, the second jaw 44 rotates around the pivot 50 and moves away from the first jaw 42. In other words, assume here that the second jaw 44 moves close to and away from the first jaw 42 through an appropriate operation of the operation section 24. The structure in which the second jaw 44 moves close to and away from the first jaw 42, is well known and thus its description will be omitted.

It is also favorable that the known structure allows the first jaw 42 to rotate at the distal end of the sheath 34, allows the second jaw 44 to rotate at the distal end of the sheath 34, and allows the first and second jaws 42 and 44 to simultaneously move close to and away from each other.

It is preferable that the first and second jaws 42 and 44 be formed of a rigid material such as a stainless alloy. It is preferable that the outer surfaces of the first and second jaws 42 and 44 be electrically insulated. It is preferable that like the first jaw 42, the sheath 34 is cylindrically formed of a rigid material such as a stainless alloy material and its surface be electrically insulated.

The blade 46 is formed of a material having good heat conductivity, such as a copper alloy material and an aluminum alloy material. It is preferable that the blade rest 48 be formed of a material having electrical insulating properties and heat-resisting properties, such as PTFE. It is also preferable that the blade rest 48 is formed such that it can be deformed elastically as appropriate by pressure applied from a biological tissue LT when the biological tissue LT is grasped between the blade 46 and the blade rest 48.

The blade 46 in the first jaw 42 is located close to the second jaw 44. The blade rest 48 in the second jaw 44 is located close to the first jaw 42. Thus, when the second jaw 44 moves close to the first jaw 42, the blade rest 48 moves close to the blade 46. When the second jaw 44 moves away from the first jaw 42, the blade rest 48 moves away from the blade 46.

A heater 56 is disposed as a heat source between the first jaw 42 and the blade 46. Accordingly, the energy source 14a of the controller 14 is controlled, and the energy source 14a supplies appropriate energy to the heater 56 to heat the blade 46. In other words, the heat transferred from the heater 56 increases the temperature of the blade 46 from the distal end to the proximal end of the top portion 46a over a cutting area 64 from a sealing area 62.

It is favorable that the heater 56 is not disposed between the first jaw 42 and the blade 46 but the heater 56 is embedded in the blade 46.

A heat insulation material 58 having heat-resisting properties against temperatures exceeding, e.g. 300°C and low heat conductivity, is disposed between the first jaw 42 and the heater 56. The heat insulation material 58 in which the heat-resisting properties of the heat insulation material 58 are lower than those of the stainless alloy material such as a PTFE material, is disposed as a spacer between the first jaw 42 formed of the stainless alloy and the heater 56 to secure a distance between the first jaw 42 and the heater 56. Thus, the heater 56 is prevented from transferring heat directly to the surface of the first jaw 42.

Furthermore, it is favorable that an air layer is formed in place of the heat insulation material 58 to inhibit heat from transferring from the heater 56 to the first jaw 42. In other words, an object need not necessarily be disposed between the heater 56 and the first jaw 42.

As shown in FIGS. 2 to 4, the blade 46 extends from its distal end to proximal end and has the top portion 46a including a ridge at a position opposed to the blade rest 48 of the second jaw 44. The blade 46 has a longitudinal axis L extending between the distal-end portion (sealing area 62) and the proximal-end portion (cutting area 64) . The longitudinal axis L of the blade 46 extends along the top portion 46a. The top portion 46a extends continuously in parallel to or substantially in parallel to the longitudinal axis L. It is thus preferable that when the second treatment piece 54 is closed relative to the first treatment piece 52 and the top portion 46a of the blade 46 is moved close to the grasping section 48a of the blade rest 48, the top portion 46a of the blade 46 be brought into contact with almost all the grasping section 48a of the blade rest 48 from its distal end to proximal end.

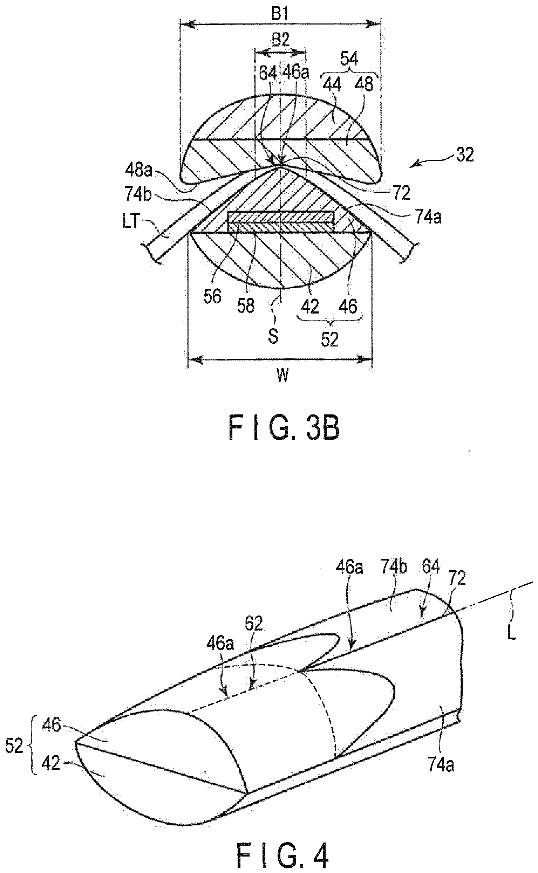

It is preferable that the blade 46 is formed such that its length along the longitudinal axis L is greater than the width W in the width direction orthogonal to the longitudinal axis L. It is also preferable that the blade 46 is formed such that its cross section is symmetrical with regard to a virtual surface S along the opening and closing direction of the second jaw 44 including, e.g. the longitudinal axis L, as shown in FIGS. 3A and 3B.

The blade 46 includes a sealing area (first pressure applying section) 62 to grasp a biological tissue LT in cooperation with the grasping section 48a of the blade rest 48 and to seal the biological tissue LT in cooperation with the grasping section 48a by applying energy thereto, at the distal-end portion along the longitudinal axis L. The blade 46 includes a cutting area (second pressure applying section) 64 formed to continue to a proximal side of the sealing area 62 along the longitudinal axis L. The cutting area 64 grasps the biological tissue LT in cooperation with the grasping section 48a and cuts the biological tissue while the cutting area 64 seals the biological tissue LT in cooperation with the grasping section 48a by the application of energy. Thus, the cutting area 64 to cut the biological tissue LT is formed from the proximal end of the sealing area 62 to the proximal-end portion of the blade 46.

The length of the blade 46 is, for example, about 18 mm to 20 mm. The length of the sealing area 62 along the longitudinal axis L in the blade 46 is shorter than the length of the cutting area 64 along the longitudinal axis L. In other words, the length of the distal-end portion (sealing area 62) along its longitudinal axis L in the blade 46 is smaller than the length between the proximal end of the distal-end portion (sealing area 62) and the proximal end of the proximal-end portion (cutting area 64) along the longitudinal axis L. It is preferable that the length of the sealing area 62 along the longitudinal axis L in the blade 46 is about 0.5 mm through 2.5 mm.

The treatment section 32 and sheath 34 as a whole are formed to allow a trocar (not shown) having an appropriate inner diameter to be inserted therethrough. The width W of the blade 46 is, for example, about 4 mm through 5 mm. It is assumed in this embodiment that the trocar is inserted through the treatment section 32 and the sheath 34, but the treatment section 32 or the sheath 34 need not be formed to allow the trocar to be inserted therethrough. For example, it is noted that the treatment section 32 and the sheath 34 can be modified appropriately as scissor-type equipment used in an abdominal operation without a trocar. In this case, the grasping section 48a is provided at the distal-end portion of the treatment section 32, a handle is provided at the proximal-end portion of the treatment section 32, and an intermediate portion of the treatment section 32 is provided pivotably relative to the sheath 34 through the pivot 50.

The cutting area 64 includes a cutting section (edge portion) 72 to cut the biological tissue LT and sealing sections 74a and 74b to seal the biological tissue LT. The top portion 46a of the sealing area 62 in the distal-end portion of the blade 46 and the top portion 46a of the cutting area 64 on the proximal-end side of the sealing area 62 are present on the longitudinal axis L. In the cutting area 64, the cutting section 72 is present on the top portion 46a. The top portion 46a is a ridge extending along the longitudinal axis L. The top of the top portion 46a forms a ridge line extending along the longitudinal axis L, as can be seen from FIGS. 3A and 3B. In the cutting area 64, the ridge line becomes the cutting section 72.

The radius of curvature Rd of the top portion 46a in the distal-end portion of the sealing area 62, namely the blade 46 is greater than the radius of curvature Rp of the top portion 46a in the proximal-end portion of the cutting area 64, namely the blade 46. It is preferable that the distal-end portion of the blade 46, namely the top portion 46a in the sealing area 62 is formed to have an arc-shaped cross section. For this reason, the distal-end portion, namely the sealing area 62 of the blade 46 has an arc-shaped peripheral surface. The radii of curvatures Rd and Rp of the top portion 46a are formed to decrease from the distal-end portion of the blade 46 to the proximal-end side thereof. More specifically, the ridge of the sealing area 62 of the blade 46 is formed bluntly and the ridge of the cutting area 64 is formed more sharply than the sealing area 62. In other words, the ridge of the sealing area 62 of the blade 46 is formed to have an appropriate width in the width direction orthogonal to the longitudinal axis L. The ridge of the cutting area 64 of the blade 46 is formed to have a width that is considerably smaller than the width of the sealing area 62 in the width direction orthogonal to the longitudinal axis L.

The radius of curvature Rd of the distal-end portion of the blade 46 or the sealing area 62 of the blade 46 is formed such that a biological tissue LT can be coagulated (sealably) in cooperation with the grasping section 48a that faces the sealing area 62 of the blade rest 48 by applying energy (thermal energy from the heater 56 here) to the top portion 46a.

The top portion 46a, namely the cutting section (edge portion) 72 in the cutting area 64 is defined by two planar or substantially planar sealing sections 74a and 74b. The radius of curvature Rp of a section including the cutting section 72 of the cutting area 64 is so formed that a biological tissue LT can be cut in cooperation with a portion opposed to the blade rest 48 by applying energy (thermal energy from the heater 56 in this embodiment) to the top portion 46a, namely the cutting section 72. The cutting section 72 is formed as a section that is sharper than the sealing area 62. In this embodiment, the cutting section 72 is shaped like a straight line. Thus, the width of the cutting section 72 is considerably smaller than the width W of the blade 46.

The blade rest 48 includes the grasping section 48a to grasp a biological tissue LT. In this embodiment, the grasping section 48a is substantially planar. The grasping section 48a is preferably curved surface, arcuate or the like. When the second jaw 44 is close to the first jaw 42, the blade 46 moves close to the blade rest 48 and the grasping section 48a contacts the top portion 46a of the blade 46.

The energy source 14a of the controller 14 is electrically connected to the heater 56 through the operation section main body 26 of the operation section 24 and the sheath 34 of the insertion section 22. The controller 14 includes a switch unit 16. Or the controller 14 is connected to the switch unit. The switch unit 16 includes, for example, a foot switch shown in FIG. 1 and a hand switch (not shown) provided on the operation section 24. When a push pad 16a of the foot switch 16 shown in FIG. 1 is pushed, the energy source 14a supplies the heater 56 with energy that is controlled appropriately by the controller 14 to increase the heater 56 to an appropriate temperature. The controller 14 controls the energy source 14a to increase the temperature of the heater 56, for example, from about 200°C to 300°C in a few seconds after when the push pad 16a of the switch unit 16 is pushed.

Next is a description of the operation of the treatment system 10 according to this embodiment.

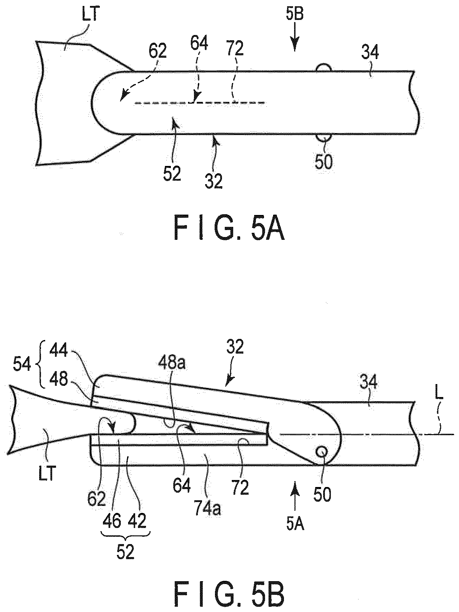

As shown in FIGS. 5A to 6, the second jaw 44 is operated as appropriate relative to the first jaw 42 to grasp a biological tissue LT by the distal-end portion of the treatment section 32. Specifically, the biological tissue LT is grasped in cooperation between the sealing area 62 of the blade 46 and the grasping section 48a of the blade rest 48. Then, the biological tissue LT is grasped by a plane defined by the length of the sealing area 62 along the longitudinal axis L and the appropriate width of the top portion 46a that is orthogonal to the longitudinal axis L. The sealing area 62 is formed as a curved surface shaped like an arc or the like. An appropriate pressure is therefore applied to the biological tissue LT grasped between the sealing area 62 and the grasping section 48a of the blade rest 48. In this embodiment, the blade 46 applies the highest pressure to the biological tissue LT in the middle in the width direction. The sealing area 62 of the blade 46 will not apply such a pressure as to cut the biological tissue LT to the biological tissue LT.

The length of the sealing area 62 along the longitudinal axis L is, for example, about 2 mm. The width of the top portion 46a that contacts the biological tissue LT in the sealing area 62 is, for example, about 1 mm through several mm (which is less than the maximum width W of the blade 46). Thus, the contact section between the biological tissue LT and the sealing area 62 of the blade 46 becomes planar. If, therefore, the operation section 24, namely the treatment instrument 12 is moved while the biological tissue LT between the sealing area 62 in the distal-end portion of the blade 46 and the grasping section 48a of the blade rest 48 is grasped, the biological tissue LT can be pulled.

Then, as described above, the biological tissue LT between the sealing area 62 in the distal-end portion of the blade 46 and the grasping section 48a of the blade rest 48 is grasped not locally but planarly. Thus, when the biological tissue LT is first grasped between the sealing area 62 and the grasping section 48a of the blade rest 48 and then the grasped biological tissue LT is released, it is prevented from being damaged as much as possible, such as that a mark of the grasping will be left and the grasped portion will undergo necrosis.

When the energy source 14a supplies energy to the heater 56 with the biological tissue LT grasped between the sealing area 62 and the grasping section 48a of the blade rest 48, the heater 56 generates heat. At this time, the temperature of the heater 56 increases to, for example, about 200°C to 300°C from room temperature in a few seconds by energy output from the energy source 14a while the heater 56 is controlled by the controller 14. Accordingly, the heat is transferred from the heater 56 toward the top portion 46a of the blade 46. At this time, the biological tissue LT is planarly in contact with the sealing area 62 and thus the biological tissue LT is heated planarly. Since the biological tissue LT is heated planarly at the top portion 46a of the sealing area 62, the heated section is planarly sealed (coagulated). The controller 14 performs control such that the output of energy from the energy source 14a to the heater 56 is stopped in a few seconds after the temperature of the heater 56 increases to, for example, about 200°C to 300°C.

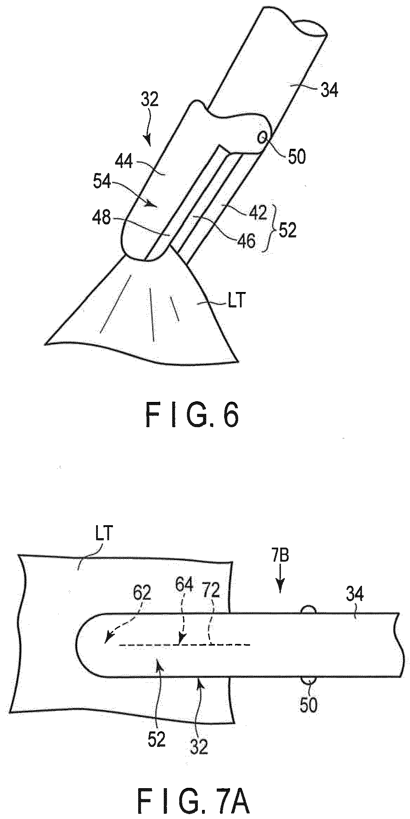

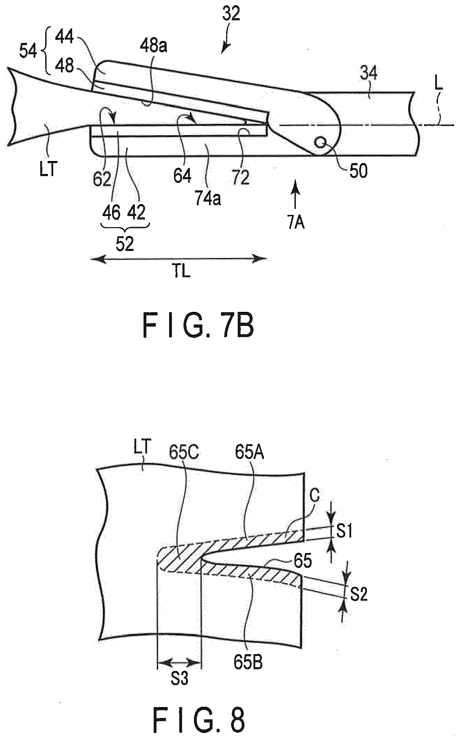

To cut the biological tissue LT, as shown in FIGS. 7A and 7B, while the biological tissue LT is grasped by the grasping section 48a of the blade rest 48 opposed to the sealing area 62 and the sealing area 62, it is grasped by the grasping section 48a of the blade rest 48 opposed to the cutting area 64 and the cutting area 64 formed continuously to the sealing area 62. When energy is supplied to the heater 56 from the energy source 14a in this state, the biological tissue LT between the sealing area 62 and the grasping section 48a of the blade rest 48 is coagulated as described above.

The cutting section 72 of the cutting area 64 is formed as a portion that is sharper than the sealing area 62. In the cutting area 64, therefore, a greater pressure is applied to a portion of the biological tissue LT which is in contact with the cutting section 72 than a portion thereof which is in contact with the sealing sections 74a and 74b.

When energy is supplied to the heater 56 from the energy source 14a with the biological tissue LT grasped between the cutting area 64 and the grasping section 48a of the blade rest 48, the heater 56 transfers heat to the cutting section 72 of the top portion 46a of the blade 46. Heat is transferred to substantially planar sealing sections 74a and 74b to form the cutting section 72 as well as the cutting section 72. Accordingly, heat is also transferred to sealing sections 74a and 74b that are close to the cutting section 72.

In the cutting area 64, a portion of the biological tissue LT which is in contact with the cutting section 72 is cut by the cutting section 72 by local pressure and heat, and a portion thereof which is in contact with the sealing sections 74a and 74b adjacent to the cutting section 72 is sealed by heat. For this reason, in the cutting area 64, the biological tissue LT is cut and sealed, or coagulated almost at the same time. In the cutting area 64, therefore, the biological tissue LT is cut while being sealed (coagulated) without bleeding. Accordingly, a cut area 65 is formed in a section interposed between the cutting section 72 of the blade 46 and the proximal-end portion of the blade rest 48 in the biological tissue LT. In the biological tissue LT, a section interposed between each the first and second sealing sections 74a and 74b and the proximal-end portion of the blade rest 48 is not cut but coagulated by heat. Furthermore, sealed areas (width-direction seal margins) 65A and 65B in which the biological tissue LT is sealed, are formed on both sides of the cut area 65 of the biological tissue LT in the width direction of the treatment section 32 (extending direction of the biological tissue LT) .

The distal end of the cutting area 64 coincides with the proximal end of the sealing area 62. For this reason, the biological tissue LT is cut while being sealed (coagulated) at the distal end of the cutting area 64. As shown in FIG. 8, therefore, when the biological tissue LT is cut, a substantially U-shaped sealed area C is formed in the biological tissue LT. Thus, a sealed area (distal-end seal margin) 65C in which the biological tissue LT is sealed, is formed on the distal-end side of the cut area 65 of the biological tissue LT in the longitudinal direction (biological tissue LT) of the treatment section 32.

To extend the cut area 65 of the biological tissue LT shown in FIG. 8 to be separated into two tissues, the second jaw 44 is opened relative to the first jaw 42 to advance the treatment section 32 to the biological tissue LT along the longitudinal axis L. While the biological tissue LT is grasped by the grasping section 48a of the blade rest 48 opposed to the sealing area 62 and the sealing area 62, it is grasped by the grasping section 48a of the blade rest 48 opposed to the cutting area 64 and the cutting area 64 formed continuously to the sealing area 62. Then, the biological tissue LT is cut while being sealed (coagulated) as described above. Thus, the biological tissue LT is cut while being sealed to be separated into two tissues.

Below is a description of a treatment to be performed by grasping a blood vessel V whose diameter (thickness) is greater than the overall length TL of the second jaw 44 in its longitudinal direction as a treatment target.

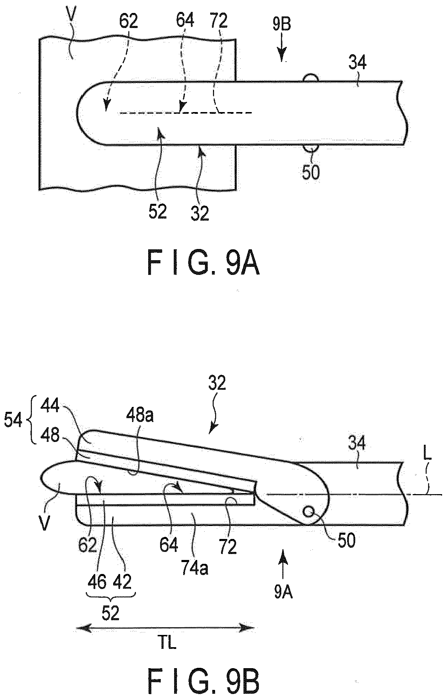

As shown in FIGS. 9A and 9B, when a blood vessel V whose diameter is greater than the overall length TL of the second jaw 44 is grasped, even though the blood vessel V is grasped between the first and second jaws 42 and 44, the blade rest 48 of the second jaw 44 cannot be brought into contact with the entire width of the blood vessel V at once in the radial direction of the blood vessel V. Thus, the blade rest 48 is brought into contact with the blood vessel V not over the entire width but in part in the radial direction of the blood vessel V with the blood vessel V grasped between the first and second jaws 42 and 44.

When there is no treatment target between the first and second jaws 42 and 44, the cutting section 72 of the blade 46 contacts the proximal-end portion of the blade rest 48. Thus, when the blood vessel V is grasped between the first and second jaws 42 and 44, the pressure acting on a section interposed between the cutting section 72 of the blade 46 and the proximal-end portion of the blade rest 48 in the blood vessel V increases. If, therefore, the blade 46 radiates heat with the blood vessel V grasped between the first and second jaws 42 and 44, a section interposed between the cutting section 72 of the blade 46 and the proximal-end portion of the blade rest 48 in the blood vessel V is cut (incised) by pressure and heat. Accordingly, the cut area 65 is formed in the section interposed between the cutting section 72 of the blade 46 and the proximal-end portion of the blade rest 48 in the blood vessel V. Furthermore, an uncut remaining part 63 is formed in a section which the distal-end portion of the blade rest 48 of the blood vessel V does not contact in the radial direction of the blood vessel V.

When the cutting section 72 of the blade 46 is in contact with the proximal-end portion of the blade rest 48, the planar first sealing section 74a and the second sealing section 74b are each separated from the proximal-end portion of the blade rest 48 and do not contact the proximal-end portion of the blade rest 48. Therefore, in the first sealing section 74a located on one side of the cutting section 72 in the width direction of the first jaw 42 and the second sealing section 74b located on the other side of the cutting section 72 in the width direction of the first jaw 42, the pressure acting on the blood vessel V between the first jaw 42 and the second jaw 44 becomes smaller than in the cutting section 72. In other words, the pressure acting on the blood vessel V in a section interposed between each of the first sealing section 74a and the second sealing section 74b and the proximal-end portion of the blade rest 48 becomes smaller than that in the section interposed between the cutting section 72 and the proximal-end portion of the blade rest 48. For this reason, the section interposed between each of the first sealing sections 74a and the second sealing section 74b and the proximal-end portion of the blade rest 48 in the blood vessel V is not cut but coagulated by heat. Accordingly, sealed areas (width-direction seal margins) 65A and 65B in which the blood vessel V is sealed are formed on both sides of the cut area 65 of the blood vessel V in the width direction of the treatment section 32 (extending direction of the blood vessel V) . Since the sealed areas 65A and 65B are formed, the cut area 65 can effectively be prevented from bleeding from both sides in the width direction of the treatment section 32 even though the blood vessel V is cut.

According to an embodiment, as shown in FIG. 3B, the dimension B1 of the second jaw 44 in its width direction is 4 mm or more and 5 mm or less, and the dimension B2 of the cutting section 72 in the width direction of the treatment section 32 is 0.5 mm or more and 1 mm or less. In this case, the dimension (corresponding one of S1 and S2) of each of the sealed areas 65A and 65B in the extending direction of the blood vessel V is, for example, 0.5 mm or more and 2.5 mm or less.

Furthermore, when the cutting section 72 of the blade 46 is in contact with the proximal-end portion of the blade rest 48, it is brought into contact with the distal-end portion of the blade rest 48. Since, however, the distal-end portion of the cutting section 72 is formed more bluntly than the proximal-end portion of the cutting section 72, the pressure acting on the blood vessel V in a section interposed between the cutting section 72 and the distal-end portion of the blade rest 48 becomes smaller than that in the section interposed between the cutting section 72 and the proximal-end portion of the blade rest 48. For this reason, the section interposed between the distal-end portion of the cutting section 72 and that of the blade rest 48 (the section close to a portion that the distal end of the cutting section 72 of the blade 46 contacts) in the blood vessel V is not cut but coagulated by heat. Thus, a sealed area (distal-end seal margin) 65C in which the blood vessel V is sealed is formed between the cut area 65 and the uncut remaining part 63 of the blood vessel V in the longitudinal direction of the treatment section 32 (the radial direction of the blood vessel V). Since the sealed area 65C is formed, bleeding from between the portion that the distal end of the cutting section 72 of the blade 46 contacts and the uncut remaining part 63 can be prevented effectively even though the blood vessel V is cut. In other words, the cut area 65 and the uncut remaining part 63 can be sealed appropriately in the radial direction of the blood vessel V.

As described above, in this embodiment, even when a blood vessel V whose diameter is large (the blade rest 48 of the second jaw 44 cannot be brought into contact with the blood vessel V at a time over the entire width thereof) is cut, the sealed area (distal-end seal margin) 65C is formed between the cut area 65 and the uncut remaining part 63 of the blood vessel V. The sealed area 65C makes it possible to prevent bleeding and ensure treatment performance and treatment efficiency in the treatment of cutting a large-diameter (thick) blood vessel V.

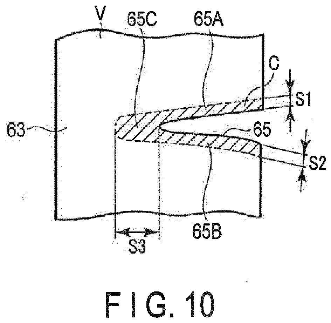

To extend the cut area 65 of the blood vessel V shown in FIG. 10 to be separated into two vessels, the second jaw 44 is opened relative to the first jaw 42 to advance the treatment section 32 to the blood vessel V along the longitudinal axis L. While the blood vessel V is grasped by the grasping section 48a of the blade rest 48 opposed to the sealing area 62 and the sealing area 62, it is grasped by the grasping section 48a of the blade rest 48 opposed to the cutting area 64 and the cutting area 64 formed continuously to the sealing area 62. Then, the blood vessel V is cut while being sealed (coagulated) as described above. Thus, the blood vessel V is cut while being sealed to be separated into two vessels.

As described above, the following can be seen from the treatment system 10 according to this embodiment, especially the treatment instrument 12 as medical apparatus.

As for the biological tissue LT that need not be treated but is grasped only, it is grasped between the sealing area 62 of the blade 46 and the grasping section 48a of the blade rest 48. In other words, the biological tissue LT can be grasped using only the distal-end portion of the treatment section 32. Then, the biological tissue LT is grasped planarly by the sealing area 62 of the blade 46 and the grasping section 48a of the blade rest 48. Thus, the treatment instrument 12 makes it possible to grasp the biological tissue LT while inhibiting a mark of the grasping from being left as much as possible when the biological tissue LT is released from the treatment section 32.

When the biological tissue LT to be treated is cut while being coagulated, it is grasped between the sealing area 62 of the blade 46 and the grasping section 48a of the blade rest 48 and between the cutting area 64 of the blade 46 and the grasping section 48a of the blade rest 48. Then, a great pressure is applied locally to the biological tissue LT between the cutting area 64 of the blade 46 and the grasping section 48a of the blade rest 48. A great pressure is particularly applied to a portion that is in contact with the cutting section 72. A section close to the portion that is in contact with the cutting section 72 is in contact with the sealing sections 74a and 74b. If, therefore, energy is supplied, the cutting area 64 of the blade 46 allows the function of the cutting section 72 to be fulfilled while exerting a sealing function of the sealing sections 74a and 74b. Accordingly, the entire treatment section 32 can perform treatment such as coagulation and incision.

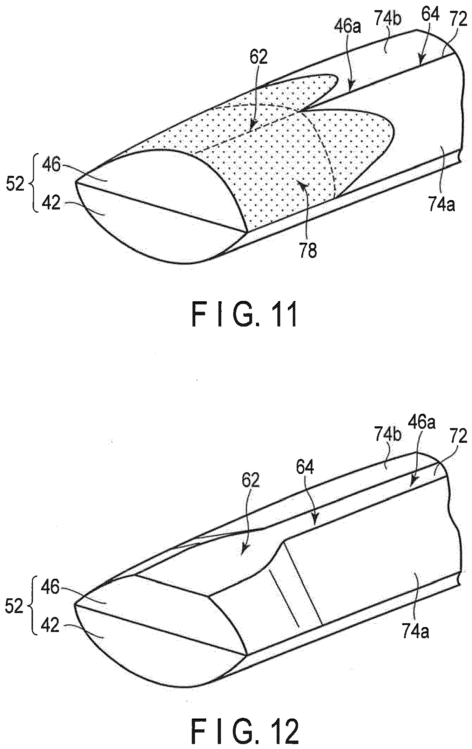

In this embodiment, an example where the cross section of the sealing area 62 of the blade 46 is shaped like a substantially arc and the surface of the sealing area 62 is smooth, has been described. As a first modification, as shown in FIG. 11, it is favorable that a non-slip portion 78 be formed in the sealing area 62 to prevent the biological tissue LT from slipping by, e.g. satin and uneven surfaces. It is also favorable that a non-slip portion (not shown) be formed in a portion of the blade rest 48, which is opposed to the sealing area 62 of the blade 46, to prevent the biological tissue LT from slipping by, e.g. satin and uneven surfaces.

The shapes of the sealing area 62 and cutting area 64 of the blade 46 is not limited to the shapes shown in FIGS. 3A to 4. As a second modification, a ridge line that extends over the cutting area 64 from the sealing area 62 may have a width as shown in FIG. 12, for example. If a ridge portion is formed by a plane surface or a curved surface near to a substantially plane surface (referred to as a plane hereinafter) and the width of the plane in the sealing area 62 is made greater than that of the plane in the cutting area 64, the ridge of the cutting area 64 becomes sharper than that of the sealing area 62, thus producing the same advantage as when they take the shapes shown in FIGS. 3A to 4. It should be noted that various combinations can be changed as appropriate, for example, the ridge portion of the sealing area 62 is shaped like a plane and the edge of the cutting area 64 is shaped as shown in FIG. 3B or the ridge portion of the cutting area 64 is shaped like a plane and the ridge of the sealing area 62 is shaped as shown in FIG. 3A.

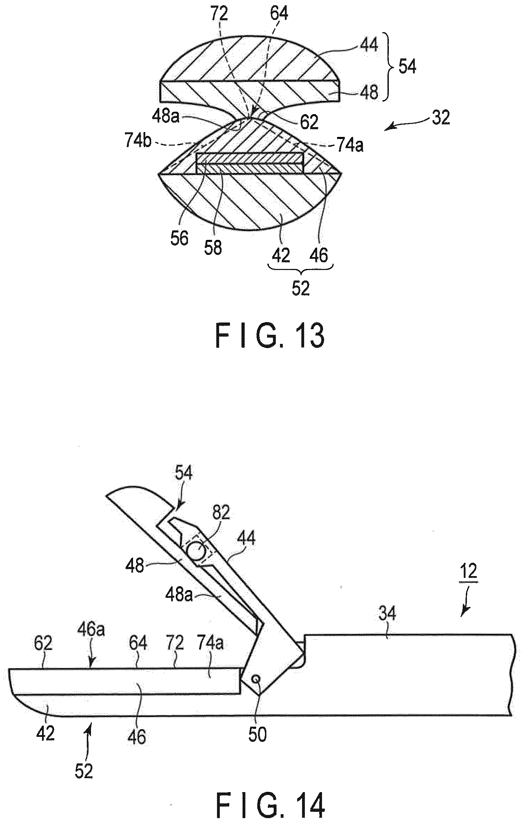

As a third modification, the blade rest 48 includes a convex grasping section 48a extending along the longitudinal axis L, as shown in FIG. 13. In other words, the grasping section 48a of the blade rest 48 need not be formed as a plane.

As a fourth modification, it is favorable that the second treatment piece 54 be formed such that the blade rest 48 is supported rotatably with regard to the second jaw 44 by a support pin 82, as shown in FIG. 14. In other words, it is favorable that the second treatment piece 54 be also formed as a so-called seesaw jaw. If the biological tissue LT is grasped while the blade rest 48 is rotating appropriately with regard to the second jaw 44, the grasping pressure of the biological tissue LT between the blade 46 and the blade rest 48 can be uniformed. Though not shown, it is favorable that the first treatment piece 52 be also formed as a so-called seesaw jaw.

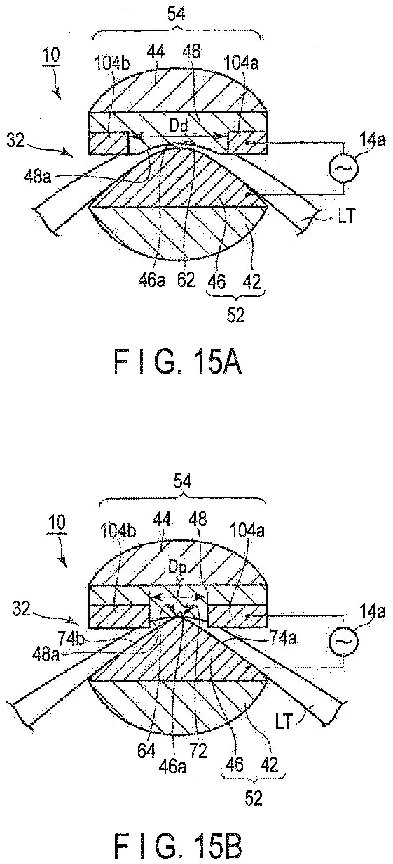

A second embodiment will now be described with reference to FIGS. 15A to 16. This embodiment is a modification to the first embodiment including the modifications, and the members having the same member or the same function as the members described in the first embodiment are denoted by the same sign as much as possible and their detailed descriptions will be omitted. In the second embodiment, an example of using high-frequency energy in place of thermal energy of the heater 56 will be described.

The blade 46 of the first treatment piece 52 shown in FIGS. 15A and 15B has conductivity and functions as a first high-frequency electrode. The blade rest 48 of the second treatment piece 54 is provided with a pair of second high-frequency electrode 104a and 104b. The blade rest 48 has electrical insulation properties. The second high-frequency electrodes 104a and 104b are electrically connected to each other, e.g. at the proximal-end portion sections of the treatment section 32. Accordingly, high-frequency energy is output between the blade (first high-frequency electrode) 46 and the second high-frequency electrodes 104a and 104b. The blade 46 and the second high-frequency electrode 104a and 104b can be formed of the same material, and favorably, they can be formed using materials having different characteristics, such as a copper alloy and an aluminum alloy.

The edge portion of the grasping section 48a of the blade rest 48 is formed flush with the opposing edge portions of the second high-frequency electrodes 104a and 104b. The widthwise middle portion of the grasping section 48a is formed like a curved surface with which the sealing area 62 of the top portion 46a of the blade 46 is brought into contact like an arc with respect to the opposing edge portions of the second high-frequency electrodes 104a and 104b. The area of contact between the sealing area 62 of the blade 46 and the grasping section 48a of the blade rest 48 can thus be increased.

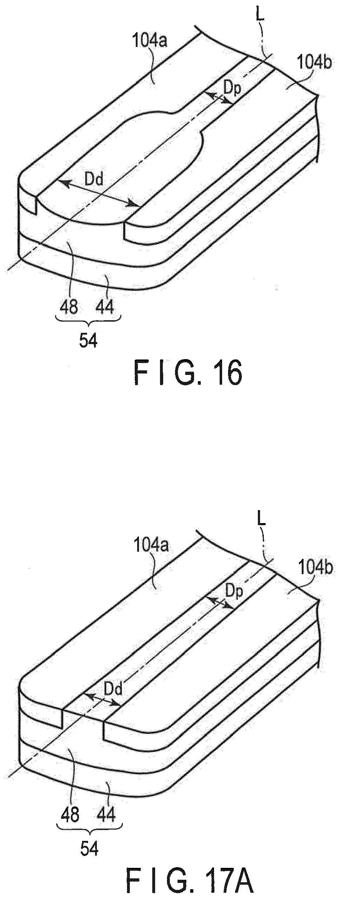

As shown in FIG. 16, the second high-frequency electrodes 104a and 104b of the second treatment piece 54 are arranged separately from the blade rest 48 in the width direction orthogonal to the longitudinal axis L so as not to contact the blade 46 of the first treatment piece 52 with the second treatment piece 54 closed relative to the first treatment piece 52. The surfaces of the second high-frequency electrodes 104a and 104b of the second treatment piece 54 are projected toward the first treatment piece 52 more slightly than the grasping section 48a of the blade rest 48. When the second treatment piece 54 is closed relative to the first treatment piece 52, the blade 46 of the first treatment piece 52 is prevented from being brought into contact with the second high-frequency electrodes 104a and 104b. Specifically, in the blade rest 48, the distance Dd between the second high-frequency electrodes 104a and 104b located opposite to the sealing area 62 is greater than the distance Dp between the second high-frequency electrodes 104a and 104b located opposite to the cutting area 64. In other words, between the paired second high-frequency electrode 104a and 104b, the distance Dd in a section opposed to the sealing area (distal-end portion) 62 of the top portion 46a is greater than the distance Dp in a section opposed to the cutting area (proximal-end portion) 64 of the top portion 46a. Then, the longitudinal axis L is located at the midpoint of the distance Dd and the midpoint of the distance Dp with the second treatment piece 54 closed relative to the first treatment piece 52. Therefore, when the top portion 46a of the blade 46 is brought into contact with the blade rest 48 as shown in FIGS. 15A and 15B, the distance between the blade 46 and the second high-frequency electrodes 104a and 104b can be reduced while the blade 46, namely the first high-frequency electrode and the second high-frequency electrodes 104a and 104b are separated from each other. The biological tissue LT can easily be brought into contact with the blade 46 and both the second high-frequency electrodes 104a and 104b of the blade rest 48 with the second treatment piece 54 closed relative to the first treatment piece 52.

The energy source 14a is electrically connected to the blade 46, namely the first high-frequency electrode and each of the second high-frequency electrodes 104a and 104b of the blade rest 48. Thus, when the energy source 14a supplies energy with the biological tissue LT between the blade 46 and the second high-frequency electrodes 104a and 104b of the blade rest 48, the biological tissue LT can be treated by the Joule heat.

At that time, the biological tissue LT is planarly in contact with the sealing area 62 including the top portion 46a of the blade 46. As has been described in the first embodiment, the temperature of the biological tissue LT grasped between the sealing area 62 of the blade 46 and the grasping section 48a of the blade rest 48 is increased planarly by the action of high-frequency energy. Since the temperature of the biological tissue LT is increased planarly at the top portion 46a of the sealing area 62, a section that has increased in temperature is sealed (coagulated) planarly.

Moreover, the biological tissue LT is in contact with the cutting area 64 including the top portion 46a of the blade 46. At this time, the biological tissue LT is in contact with at least the cutting section 72 and a portion of the sealing sections 74a and 74b which is close to the cutting section 72. As has been described in the first embodiment, therefore, in the cutting area 64, a portion of the biological tissue LT which is in contact with the cutting section 72 is cut by the cutting section 72 by the local pressure and the Joule heat caused by high-frequency energy, and a portion thereof which is in contact with the sealing sections 74a and 74b adjacent to the cutting section 72 is sealed by the Joule heat. For this reason, the cutting and the sealing or coagulation of the biological tissue LT are performed almost at the same time in the cutting area 64. In the cutting area 64, therefore, the biological tissue LT is cut while being sealed (coagulated) without bleeding.

A method of supplying energy from the energy source 14a to the second high-frequency electrodes 104a and 104b of the blade rest 48 of the blade 46 can appropriately be set. The energy source 14a is able to supply energy appropriately to the blade 46 and the second high-frequency electrodes 104a and 104b of the blade rest 48 and stop supplying the energy in accordance with, for example, an impedance change of the biological tissue LT between the blade 46 and the second high-frequency electrodes 104a and 104b of the blade rest 48.

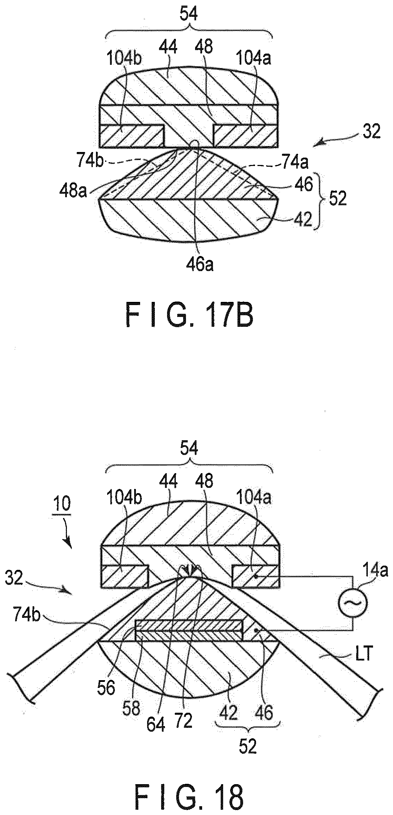

As shown in FIGS. 17A and 17B, the grasping section 48a of the blade rest 48 and the second high-frequency electrodes 104a and 104b are formed flush with each other. Thus, when the second treatment piece 54 is closed relative to the first treatment piece 52, the blade 46 of the first treatment piece 52 is prevented from being in contact with the second high-frequency electrodes 104a and 104b. Even though the grasping section 48a of the blade rest 48 is protruded to the surface of the second high-frequency electrodes 104a and 104b, the same advantage can be obtained.

It is favorable that in the blade rest 48, the distance Dd between the second high-frequency electrodes 104a and 104b located opposite to the sealing area 62 and the distance Dp between the second high-frequency electrodes 104a and 104b located opposite to the cutting area 64 be the same as shown in FIG. 17A.

A third embodiment will now be described with reference to FIG. 18. This embodiment is a modification to the first and second embodiments including the modifications, and the members having the same member or the same function as the members described in the first and second embodiments are denoted by the same sign as much as possible and their detailed descriptions will be omitted.

In the first embodiment, an example of cutting the biological tissue LT while being sealed by heat transfer from the heater 56 to the blade 46 was described. In the second embodiment, an example of cutting the biological tissue LT while being sealed using high-frequency energy was described. As shown in FIG. 16, in this embodiment, the first treatment piece 52 is formed so as to be heated by heat from the heater 56 using the blade 46 as a high-frequency electrode.

In this case, the controller 14 controls the energy source 14a to apply energy appropriately to the biological tissue LT and thus allow the biological tissue LT to be cut while being sealed. Even though no energy is applied to the biological tissue LT, the biological tissue LT can be pulled and the like by grasping the biological tissue LT between the sealing area 62 and the grasping section 48a of the blade rest 48 opposed to the sealing area 62.

In the foregoing first embodiment, an example of treating a biological tissue using thermal energy generated by the heater 56 was described. In the second embodiment, an example of treating a biological tissue using high-frequency energy as a kind of thermal energy generated by the high-frequency electrodes 46, 104a and 104b was described. In the third embodiment, an example of using both of the heater 56 and the high-frequency electrodes 46, 104a and 104b was described. When a biological tissue is treated using the high-frequency electrodes 46, 104a and 104b described in the second embodiment, the maximum temperature often becomes lower than the temperature at which a biological tissue is treated using the heater 56 described in the first embodiment. Therefore, the radius of curvature Rp of the cutting area 64 can particularly be set as appropriate according to whether energy, namely the heater 56 is used or the electrodes 104a and 104b are used.

While several embodiments have been specifically described so far with reference to the drawings, this invention is not limited to the embodiments described above, and encompasses all embodiments to be implemented without departing from the sprit thereof.

## Claims

1. A medical instrument comprising:
a jaw which is provided with a grasping section; and
a blade which is movable close to and away from the grasping section and which extends from a distal-end portion thereof to a proximal-end portion thereof, the blade having a top portion including a ridge opposed to the grasping section, and the ridge of the top portion in the proximal-end portion being sharper than the ridge of the top portion in the distal-end portion.

2. The medical instrument according to claim 1, wherein a radius of curvature of the top portion in the distal-end portion is greater than a radius of curvature of the top portion in the proximal-end portion.

3. The medical instrument according to claim 1, wherein:
the blade has a longitudinal axis extending between the distal-end portion and the proximal-end portion thereof; and
a length of the blade along the longitudinal axis in the distal-end portion is shorter than a length thereof along the longitudinal axis between a proximal end of the distal-end portion and a proximal end of the proximal-end portion.

4. The medical instrument according to claim 3, wherein the length of the blade along the longitudinal axis in the distal-end portion is 0.5 mm through 2.5 mm.

5. The medical instrument according to claim 1, wherein:
the blade has a longitudinal axis extending between the distal-end portion and the proximal-end portion thereof; and
the top portion continuously extends substantially in parallel to the longitudinal axis.

6. The medical instrument according to claim 1, wherein the grasping section is allowed to be in contact with the top portion when the blade is close to the grasping section.

7. The medical instrument according to claim 6, wherein the grasping section is provided with a pair of high-frequency electrodes.

8. The medical instrument according to claim 7, wherein a portion of the top portion opposed to the distal-end portion is longer than a portion of the top portion opposed to the proximal end portion between the pair of high-frequency electrodes.

9. The medical instrument according to claim 1, wherein a radius of curvature of the top portion becomes smaller toward a proximal-side from the distal-end portion.

10. The medical instrument according to claim 1, wherein the blade includes a sealing area which is formed at the distal-end portion, which is configured to grasp a biological tissue in cooperation with the grasping section, and which is configured to seal the biological tissue in cooperation with the grasping section by applying energy thereto, and a cutting area which is formed continuously to the sealing area and on a proximal-end side of the sealing area, which is configured to grasp a biological tissue in cooperation with the grasping section and which is configured to cut the biological tissue while the cutting area is configured to seal the biological tissue in cooperation with the grasping section by applying energy thereto.

11. The medical instrument according to claim 1, wherein the top portion has an arc-shaped peripheral surface in the distal-end portion.

12. The medical instrument according to claim 1, wherein a radius of curvature of the proximal-end portion of the blade is formed such that a biological tissue is cut while the cutting area is configured to seal the biological tissue in cooperation with the grasping section by applying energy to the top portion.

13. The medical instrument according to claim 1, wherein a radius of curvature of the distal-end portion of the blade is formed such that the distal-end portion of the blade is configured to coagulate a biological tissue in cooperation with the grasping section by applying energy to the top portion.

14. The medical instrument according to claim 1, wherein the top portion of the blade is increased in temperature from the distal-end portion of the top portion to the proximal-end portion thereof by heat transfer from a heat source.

15. The medical instrument according to claim 1, wherein:
the grasping section is provided with an electrode, and
high-frequency energy is output between the blade and the electrode.
